# NOUVEAU FASCICULE DE BREVET EUROPEEN

(11) **EP 0 115 286 B2**
(45) Date de publication et mention de la décision concernant l'opposition: **30.11.1994**
(45) Mention de la délivrance du brevet: 26.11.1986
(21) Numéro de dépôt: 84100320.5
(22) Date de dépôt: 13.01.1984
(51) Int. Cl.: A61F 13/15

(54) **Procédé de fabrication de couches-culottes à jeter et couches-culottes obtenues**
Verfahren zur Herstellung von Wegwerfwindeln und so hergestellte Wegwerfwindeln
Method of making disposable diapers and diapers produced by this method

(30) Priorité: 19.01.1983 FR 8300780
(43) Date de publication de la demande: 08.08.1984
(73) Titulaire: BOUSSAC SAINT FRERES B.S.F. Société anonyme dite:, F-59800 Lille (FR)
(72) Inventeur: De Jonckheere, Raphael, F-59910 Bondues (FR)
(74) Mandataire: Casalonga, Axel

(56) Documents cités:
- EP-A- 0 048 010
- EP-A- 0 080 647
- EP-A- 0 095 034
- DE-A- 1 943 211
- DE-C- 703 021
- FR-A- 2 412 276
- FR-A- 2 462 112
- GB-A- 2 078 811
- GB-A- 2 078 811
- US-A- 3 860 003
- US-A- 4 050 462
- US-A- 4 081 301
- US-A- 4 300 562
- US-A- 4 333 782
- US-A- 4 352 355

## Description

La présente invention a pour objet un procédé de fabrication de couches-culottes à jeter ainsi que les couches-culottes obtenues. On sait que les couches-culottes qui sont utilisées généralement pour les enfants en bas-âge mais aussi pour les adultes incontinents, comprennent sous forme intégrée à titre d'éléments principaux une enveloppe extérieure imperméable souple jouant le rôle de culotte et un matelas absorbant fixé à l'intérieur de l'enveloppe imperméable éventuellement recouvert d'un voile interne perméable à l'humidité. Une telle couche-culotte que l'on appelle parfois change complet permet de remplacer à la fois la couche absorbante traditionnelle et la culotte imperméable qui était utilisée en combinaison avec ladite couche.

On a déjà prévu de disposer des éléments élastiques le long des échancrures permettant le passage des jambes, pratiquées dans l'enveloppe extérieure imperméable jouant le rôle de culotte. Des exemples de telles couches-culottes munies d'élastiques à l'entrejambe sont décrits dans le document GB-A-2 078 811 et dans les brevets français FR-A-1.600.732 et FR-A-2.063.794 par exemple. On peut également citer les couches-culottes décrites dans les brevets américains US-A-3.860.003 et US-A-4.050.462.

En général les élastiques rectilignes sont fixés à l'enveloppe imperméable par collage sur au moins une partie de leur longueur. On utilise habituellement des bandelettes élastiques qui sont alimentées en continu à l'état tendu en étant en outre maintenues à plat c'est-à-dire avec l'une de leurs faces principales sensiblement horizontale parallèle à la surface de la feuille souple imperméable également alimentée en continu sur laquelle ils doivent être fixés. On dépose sur cette face des bandelettes élastiques, par intermittence, un matériau adhésif à température élevée en fusion (hot melt) en utilisant par exemple des moyens d'extrusion comportant un ou plusieurs gicleurs d'adhésif liquide dont le fonctionnement peut être par exemple commandé par air comprimé sous contrôle d'une électrovanne.

Compte tenu du fait que les couches-culottes ainsi fabriquées sont destinées à être jetées après usage, toute réduction même faible des coûts de fabrication et de matière première-est hautement désirée.

Par ailleurs, il est important dans les couches-culottes munies de tels moyens élastiques au voisinage de l'entrejambe que les éléments élastiques d'entrejambe n'exercent pas un serrage trop important et n'entraînent pas la formation de replis trop importants du matériau et notamment du matériau constituant le coussin absorbant afin de respecter l'anatomie de l'utilisateur.

La demande de brevet européen EP-A-00 95 034 bénéficiant d'une date de priorité du 14 avril 1982 mais publiée seulement le 30 novembre 1983, décrit un procédé de fabrication de couches-culottes à jeter utilisant un ruban élastique multibrins pour fermer les elements élastiques d'entrejambe. Toutefois la séparation des brins individuels et leur collage ne donnent pas entière satisfaction.

L'invention a pour objet principal de diminuer notablement les couts de la matière première utilisée pour la réalisation des moyens élastiques d'entrejambe tout en facilitant leur fixation par collage et en réduisant le serrage exercé sur l'utilisateur.

L'invention a également pour objet d'améliorer l'aspect esthétique de la couche-culotte en formant des fronces à l'entrejambe.

Le procédé de fabrication en continu de couches-culottes à jeter comprenant un coussin absorbant posé entre une enveloppe extérieure imperméable à l'humidité et un voile intérieur perméable, l'ensemble comportant des découpes latérales pour permettre le passage des jambes et des moyens élastiques rectilignes fixés à l'enveloppe imperméable par collage sur au moins une partie de leur longueur et disposés de chaque côté du coussin absorbant, tel qu'il est revendiqué consiste à alimenter en continu les moyens élastiques, à les, encoller de manière intermittente et les mettre en contact à l'état tendu avec la face interne de l'enveloppe extérieure imperméable entrainée par un tambour rotatif à surface lisse. Selon l'invention, on alimente en continu avec une tension prédéterminée un ruban élastique multibrins constitué par extrusion à chaud de brins individuels et solidarisation ultérieure des brins dans un seul plan par contact avant refroidissement; on sépare le ruban élastique multibrins en les différents brins individuels qui le constituent en le faisant passer de chaque côté de plusieurs tiges verticales successives de séparation disposées de manière orthogonale au trajet du ruban ; on fait passer chaque brin dans une gorge longitudinale supérieure d'un dispositif applicateur d'adhésif, ladite gorge étant alimentée par intermittence en adhésif liquide à haute température et disposée de façon que le brin passant dans la gorge soit noyé sur toute sa périphérie dans l'adhésif ; et on fait entrer en contact chaque brin ainsi enrobe d'adhésif par intermittence et tendu, avec l'enveloppe imperméable se déplaçant à la périphérie du tambour.

On obtient ainsi séparation du ruban initial multibrins en une multitude de brins individuels qui présentent chacun un diamètre égal à l'épaisseur du ruban multibrins du départ. L'utilisation pour la fabrication d'un tel ruban élastique multibrins d'un latex naturel extrudé en fils de caoutchouc nous permet l'obtention de brins individuels de très grande élasticité qui peuvent subir un allongement pouvant aller jusqu'à 300%. Il est donc possible d'alimenter le ruban elastique multibrins de façon que la tension de chacun des brins individuels entraîne un allongement maximal au moment de la fixation par collage, ce qui réduit notablement la quantité totale de matière élastique utilisée.

Après la séparation de chaque brin individuel, on fait passer chacun des brins ainsi obtenus dans une gorge longitudinale d'un dispositif applicateur d'adhésif liquide à haute température (hot melt), ladite gorge étant alimentée par intermittence en adhésif liquide de façon que le brin individuel passant dans la gorge longitudinale soit noyé sur toute sa périphérie dans l'adhésif liquide. Le passage dans le dispositif applicateur d'adhésif permet donc d'obtenir pour chacun des brins élastiques individuels, un filament élastique tendu totalement enrobé d'adhésif liquide à haute température.

On met alors en contact en continu chacun des brins ainsi enrobés d'adhésif par intermittence et tendus à une tension convenable, avec la feuille mince destinée à jouer le rôle d'enveloppe imperméable pour la couche-culotte, ladite feuille se déplaçant à la périphérie d'un tambour rotatif à surface lisse. Compte tenu de la faible épaisseur de cette feuille et de la masse du tambour rotatif, la périphérie dudit tambour se trouve à une température relativement basse par rapport à la température de l'adhésif liquide. Il est d'ailleurs possible d'agir sur cette température en prévoyant par exemple des moyens de refroidissement de la périphérie du tambour dans le cas où cela s'avérerait nécessaire. De cette manière, le matériau adhésif à haute température qui enrobe chacun des brins inviduels avant le contact avec la feuille imperméable subit un refroidissement brutal dès son contact avec ladite feuille imperméable ce qui entraîne un collage immédiat.

Le choix de la température du tambour rotatif peut aisément être opéré par le technicien en tenant compte des paramètres que constituent la masse du tambour, la masse de l'adhésif par unité de longueur pour chacun des brins élastiques, la température de l'adhésif immédiatement avant le contact ainsi que la nature de l'adhésif. Il convient à cet égard que la différence de température entre l'adhésif déposé à l'état liquide et la surface extérieure du tambour rotatif soit suffisante pour entraîner un effet de collage quasiment immédiat, apte à résister aux efforts auxquels le brin élastique est soumis dès sa fixation sur l'enveloppe. Dans la pratique, on constate qu'une différence de température d'au moins 20 _{°} C est nécessaire et de préférence de 70 à 130°C.

Dans un mode de réalisation préféré de l'invention, le ruban élastique multibrins est séparé tout d'abord en deux rubans latéraux par passage de chacun des deux rubans latéraux de chaque côté d'un organe de préséparation disposé de manière perpendiculaire au trajet du ruban. On oriente ensuite le trajet de chaque ruban latéral parallèlement au déplacement de la feuille mince destinée à former l'enveloppe extérieure de la couche-culotte, l'écartement entre les deux rubans latéraux étant adapté à l'écartement désiré pour la fixation des moyens élastiques au voisinage de l'entrajambe de chaque côté du matelas absorbant de la future couche-culotte.

La tension désirée pour les brins individuels élastiques est obtenue en alimentant le ruban élastique multibrins par un rouleau d'alimentation rotatif entraîné coopérant avec un galet de friction entrant en contact avec le rouleau, de préférence sous l'action de son propre poids. De cette manière, il est possible d'entraîner le rouleau d'alimentation rotatif de façon que la vitesse de déplacement linéaire du ruban multibrins élastique soit inférieure à la vitesse de déplacement linéaire de la feuille imperméable jouant le rôle d'enveloppe entraînée par le tambour rotatif, la différence de vitesse entraînant la tension désirée pour les élastiques.

L'alimentation du ruban multibrins peut se faire avantageusement transversalement par rapport au déplacement de l'enveloppe, des moyens de changement de direction étant alors prévus de façon que les brins individuels parviennent parallèlement au déplacement de l'enveloppe au moment de leur contact avec ladite enveloppe.

Pour résoudre la difficulté principale du procédé de l'invention, à savoir un encollage correct des différents brins individuels, il est prévu d'utiliser une installation pour la mise en oeuvre du procédé comprenant des moyens d'alimentation en continu pour l'enveloppe extérieure imperméable et pour les moyens élastiques, comprenant un dispositif applicateur d'adhésif présentant un bloc d'encollage pour chaque ensemble de brins individuels, chaque bloc d'encollage comportant un ensemble de gorges longitudinale parallèles pour chacun des ensembles de brins. Les blocs d'encollage sont disposés de façon que les brins à l'état tendu passent au-dessus dudit bloc en traversant chaque fois les gorges remplies par intermittence d'adhésif liquide.

Chaque bloc d'encollage comprend une chambre de distribution d'adhésif reliée par l'intermédiaire de passages individuels à chacune des gorges longitudinales.

L'applicateur d'adhésif comprend de préférence deux distributeurs d'adhésif coopérant avec chacun des blocs d'encollage, l'alimentation en adhésif étant commandée par un circuit d'air comprimé muni d'une électrovanne capable d'ouvrir et de fermer l'alimentation en air comprimé de manière intermittente.

La fixation du matelas absorbant et du voile perméable intérieur se fait dans le procédé de l'invention de manière en soi classique, en disposant sur la feuille imperméable une pluralité de lignes d'encollage longitudinales parallèles réparties sur toute ladite surface à l'intérieur de la feuille imperméable, ces lignes d'encollage étant réalisées par extrusion d'adhésif liquide à chaud (hot melt). Selon le procédé de l'invention, les brins élastiques individuels sont fixés par collage comme il vient d'être dit entre lesdites lignes d'encollage longitudinales de façon à se trouver enserrées comme à l'intérieur de gaines multiples entre la feuille imperméable et les autres éléments de la couche-culotte. Dans ces conditions, lors de l'étape de découpage transversal destinée à former les couches-culottes à partir d'une bande continue qui a successivement reçu les différents éléments de la couche-culotte et en particulier les matelas absorbant, les extrémités des brins élastiques non encollés reviennent sur eux-mêmes à l'intérieur de ces gaines, les parties encollées qui se trouvent au voisinage de l'entrejambe assurant au contraire la rétraction dans cette zone de la couche-culotte dans son ensemble formant ainsi des plis multiples ou fronces évitant toute fuite à cet endroit.

Selon l'invention, les différents brins élastiques individuels sont disposés de façon que le brin se trouvant le plus près de l'axe longitudinal de la couche-culotte soit très proche du bord longitudinal latéral du matelas absorbant ou même confondu avec ce bord latéral. Dans ces conditions ce brin élastique exerce son action de traction sur une zone de la couche-culotte qui présente une plus grande résistance compte tenu de la proximité du matelas absorbant que les zones latérales plus éloignées où s'exerce l'action des autres brins élastiques individuels et en particulier de ceux qui se trouvent le plus loin de l'axe de la couche-culotte.

Cette action différenciée entraîne la formation d'une convexité progressive de la couche-culotte dirigée vers l'extérieur qui forme donc une auge parfaitement adaptée à la morphologie de l'utilisateur.

Par ailleurs, le fait d'utiliser selon la présente invention à chaque passage de jambe plusieurs brins élastiques individuels disposés parallèlement permet de mieux répartir sur la cuisse la pression résultant de la traction exercée par les moyens élastiques.

La présente invention a également pour objet une couche-culotte à jeter comprenant
une enveloppe extérieure imperméable à l'humidité présentant des lignes d'encollage longitudinales parallèles réparties sur sa surface interne,
un coussin absorbant posé sur la surface interne de l'enveloppe extérieure imperméable et fixé à cette dernière par les lignes d'encollage précitées et
un voile intérieur perméable sur le coussin absorbant, également fixé à l'enveloppe imperméable par les lignes d'encollage précitées ;
l'ensemble comportant des découpes latérales pour permettre le passage des jambes et,
de chaque côté du coussin absorbant, plusieurs moyens élastiques rectilignes, parallèles entre eux, fixés par collage à l'état tendu sur l'enveloppe imperméable. Selon l'invention, les moyens élastiques sont constitués par au moins quatre brins élastiques individuels fixés à l'enveloppe imperméable uniquement sur une partie de leur longueur qui correspond à la zone médiane de la couche-culotte, tandis que leurs extrémités ne sont pas fixées à l'enveloppe imperméable. Les brins élastiques individuels sont écartés les uns des autres de 4 à 10 mm et de préférence d'environ 6mm. Ladite partie fixée par collage des brins élastiques a été enrobée sur toute sa périphérie d'une matière adhésive et appliquée à l'état tendu avec un allongement de l'ordre de et au maximum de 300% sur l'enveloppe imperméable. Les brins élastiques sont fixés entre lesdites lignes d'encollage à l'intérieur des gaines définies entre la feuille imperméable et les autres éléments de la couche-culotte de sorte que les extrémités des brins élastiques non encollées sont rétractées à l'intérieur desdites gaines tandis que les parties encollées assurent la rétraction de la couche-culotte dans son ensemble et la formation de fronces, dans les zones d'entrejambe. Le brin le plus proche du coussin se trouve à proximité immédiate du bord latéral extérieur du coussin.

L'invention sera mieux comprise à l'étude d'un mode de réalisation particulier décrit à titre d'exemple nullement limitatif et illustré par les dessins annexés sur lesquels :
la fig. 1 est une vue en perspective schématique d'une partie d'une installation permettant la mise en oeuvre du procédé de l'invention;
la fig. 2 est une vue en perspective partielle agrandie d'une portion d'extrémité d'un ruban élastique multibrins utilisé dans le procédé de l'invention;
la fig. 3 est une vue schématique agrandie partiellement éclatée du dispositif applicateur d'adhésif utilisé dans l'installation de la fig. I;
la fig. 4 est une vue en coupe selon IV-IV de la fig. 3 d'un des blocs d'encollage du dispositif applicateur de la fig. 3;
la fig. 5 illustre une succession d'étapes de la fabrication en continu des couches-culottes selon l'invention;
la fig. 6 est une vue de dessus avec un arraché partiel d'une couche-culotte selon l'invention à l'état étiré;
la fig. 7 est une vue en coupe transversale selon VIIVII de la fig. 6 prise dans la zone de l'entrejambe; et
la fig. 8 est une vue extérieure d'une couche-culotte selon l'invention représentée fermée et montrant la forme d'auge obtenue.

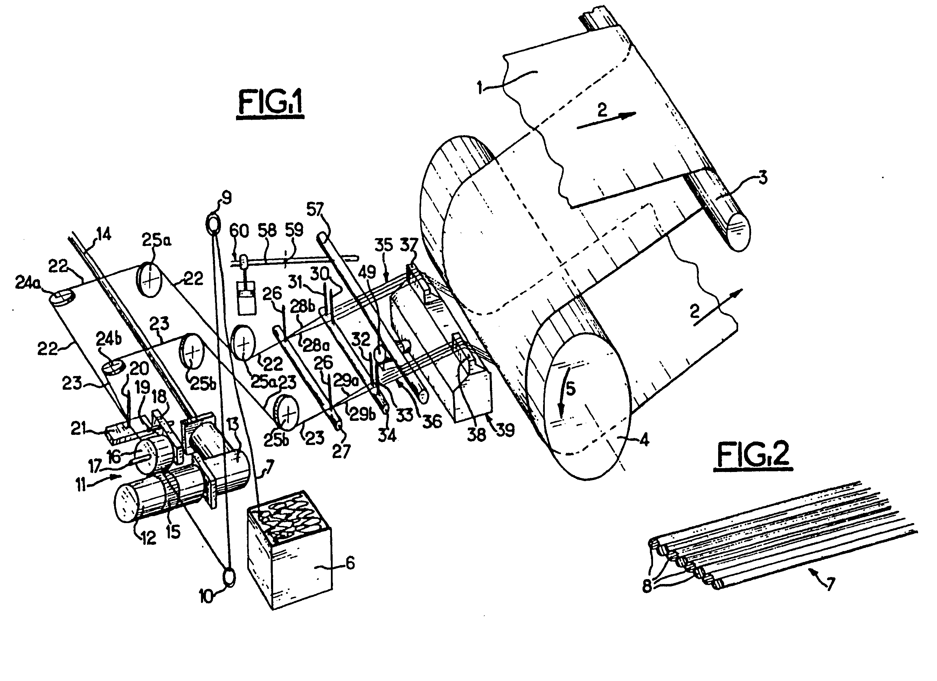

Comme on peut le voir sur la fig. 1, une feuille mince imperméable 1 est déroulée en continu dans le sens des flèches 2 entre un ensemble de rouleaux dont l'un, 3 est visible sur la figure et un tambour rotatif 4 sur la surface périphérique duquel elle se trouve plaquée le long d'un trajet qui correspond sensiblement à la moitié de la circonférence du tambour 4. La feuille 1 mince et souple est réalisée en un matériau thermosoudable imperméable à l'humidité tel que du polyéthyléne. Le tambour 4 est entraîné en rotation dans le sens de la flèche 5 et sa surface extérieure est maintenue à une température relativement basse comprise entre 30 _{°} C et 100° C. Le maintien de cette température peut être obtenu par un refroidissement artificiel forcé à l'intérieur du tambour 4 ou simplement comme illustré sur la fig. 1 grêce à l'utilisation d'un tambour métallique de masse suffisante.

Sur le côté de la machine se trouve placé un réceptacle 6 à l'intérieur duquel est rangé en vrac un ruban élastique multibrins 7 dont une portion agrandie est visible sur la fig. 2. On notera qu'à l'intérieur du réceptacle 6, le ruban élastique 7 a été préalablement rangé par plis successifs de faible longueur superposés dans des plans également successifs de sorte qu'en tirant sur l'extrémité libre supérieure du ruban 7 il est possible d'extraire la totalité du ruban contenu dans le réceptacle 6 sans risquer la formation de noeuds ou d'amas qui géneraient l'alimentation.

Comme on peut le voir sur la vue agrandie de la fig. 2, le ruban multibrins 7 est constitué, dans l'exemple illustré, par l'association de huit brins individuels 8. La fabrication d'un tel ruban multibrins se fait par extrusion individuelle de chaque brin 8 réalisé en latex naturel et extrudé sous la forme d'un fil de caoutchouc nu. Dès la sortie de l'extrudeuse, on met en contact à chaud les brins individuels qui se solidarisent par simple fusion le long d'une génératrice de façon à former un ruban sensiblement plan comme on peut le voir sur la fig. 2.

Le ruban multibrins 7 issu du réceptacle 6 passe tout d'abord par un premier anneau de guidage 9 disposé sensiblement au-dessus du réceptacle 6 et de l'ensemble de la machine. Le ruban 7 redescend ensuite jusqu'à un deuxième anneau de guidage 10 avant d'être introduit dans le dispositif d'alimentation proprement dit référencé 11 dans son ensemble. Le trajet du ruban multibrins 7 entre le réceptacle 6 d'une part et les deux anneaux de guidage 9 et 10 d'autre part, permet de régulariser l'extraction du ruban 7 sans exercer sur ce dernier de tension notable. Les boucles qui pourraient encore se former à la sortie du réceptacle 6 se trouvent éliminées au cours du trajet jusqu'au premier anneau de guidage 9 ou par le passage à travers ce dernier.

Le dispositif d'alimentation Il comprend un rouleau d'alimentation rotatif 12 entraîné en rotation par l'intermédiaire d'un couple conique 13 relié par l'arbre 14 à l'entraînement général de la machine. Sur une portion de sa surface, le rouleau d'entraînement 12 comporte des stries 15 qui en augmentent la rugosité. Un galet de friction 16 pouvant tourner fou autour de son axe 17 est supporté par un levier 18 pouvant pivoter autour d'un axe fixe 19. L'ensemble est disposé légérement au-dessus et sur le côté du rouleau d'alimentation 12 de façon que le galet 16 vienne s'appuyer par son propre poids sur la portion striée 15 du rouleau 12. Le ruban élastique multibrins 7 se trouve pincé entre la partie striée 15 du rouleau rotatif entraîné 12 et le galet de friction 16. De cette manière, le ruban multibrins 7 se trouve freiné dans son déplacement linéaire par rapport à son extrémité encollée avec la feuille souple 1. D'autre part, la rotation du rouleau 12 exerce une traction sur le ruban 7 suffisante pour l'extraire du réceptacle 6. On comprendra qu'il suffit de régler convenablement la vitesse de rotation du rouleau 12 pour faire varier la tension du ruban élastique 7 entre la sortie du dispositif d'alimentation 11 et le point d'encollage sur la feuille 1.

L'alimentation du ruban élastique 7 se fait transversalement à l'axe longitudinal de la machine comme on peut le voir sur la fig. 1, ce qui présente l'avantage de diminuer l'encombrement des différents organes et de faciliter le montage du dispositif d'alimentation 11 associé au réceptacle 6 contenant le ruban élastique 7.

Dans son trajet transversal à l'axe de la machine, le ruban multibrins 7 est amené à rencontrer une tige de préséparation 20 qui est montée sur un socle fixe 21 et disposée verticalement c'est-à-dire perpendiculairement au trajet du ruban elastique 7. La tige 20 peut être constituée par une simple tige cylindrique ayant un diamètre compris entre 1 et 5 fois le diamètre de chacun des brins individuels 8 du ruban multibrins 7. La tige 20 peut être remplacée par une lame ou tout autre organe équivalent placé dans le trajet du ruban 7.

En aval de la tige de préséparation 20, le ruban élastique 7 se trouve décomposé en deux rubans latéraux 22 et 23 qui comprennent chacun quatre brins 8 et qui poursuivent un trajet horizontal jusqu'à deux galets d'orientation 24a, 24b disposés à un même écartement de l'axe longitudinal de la machine, cet écartement correspondant sensiblement à l'écartement des moyens élastiques de la couche-culotte que l'on désire obtenir.

Les rubans latéraux 22 et 23 sont ensuite déplacés en restant dans le même plan vertical et en passant sur un ensemble de deux paires de galets 25a et 25b qui les ramènent dans un plan horizontal inférieur au plan horizontal dans lequel s'était fait l'alimentation et correspondant sensiblement au niveau horizontal du point d'encollage.

Les galets 24a et 24b ont donc permis l'orientation de chaque ruban latéral 22, 23 sensiblement parallélement au déplacement de la feuille 1 et avec l'écartement désiré entre eux. A la sortie des deux derniers galets 25a et 25b on sépare encore chaque ruban latéral 22, 23 au moyen de tiges de séparation 26 montées sur une traverse fixe 27. Les tiges 26 sont identiques à la tige de préséparation 20 et sont disposées comme elle verticalement c'est-à-dire perpendiculairement au déplacement des rubans latéraux respectifs 22, 23. Elles peuvent être réalisées au moyen d'une simple tige cylindrique de même diamère que la tige 20.

En aval des tiges 26 le ruban élastique se trouve donc séparé de chaque côté de l'axe de la machine en deux groupes de deux rubans élastiques 28a, 28b et 29a, 29b dont chacun est constitué par deux brins individuels 8 toujours accolés.

Chacun de ces rubans élastiques 28a, 28b; 29a, 29b est encore une fois séparé en passant sur une tige de séparation 30, 31, 32, 33, les quatre tiges étant montées sur une traverse fixe 34.

Les quatre tiges de séparation 30 à 33 sont disposées par paire sur la traverse 34, les tiges de chacune des paires telle que 30 et 31 d'une part, 32 et 33 d'autre part étant disposées avec un écartement déterminé l'une par rapport à l'autre de façon qu'en aval des tiges de séparation 30 à 33 le ruban élastique soit transformé en deux ensembles 35 36 de quatre brins individuels 8 se trouvant à un écartement constant les uns des autres.

Les deux ensembles de brins individuels 35 et 36 passent alors chacun respectivement sur un bloc d'encollage 37, 38 faisant partie d'un dispositif applicateur d'adhésif 39. Les deux ensembles de brins 35, 36 passent au-dessus de chaque bloc d'encollage où ils reçoivent l'adhésif comme il sera expliqué plus loin et poursuivent ensuite leur trajet en étant dirigés légérement vers le bas comme visible sur la fig. 1 jusqu'au contact avec la surface extérieure de la feuille souple 1 enroulée à la périphérie du tambour rotatif 4.

Dès le contact avec la feuille 1, l'adhésif liquide qui est porté par chaque brin individuel 8 des deux ensembles 35, 36 se trouve figé presque instantanément en raison de la différence de température entre l'adhésif liquide déposé à chaud par les blocs d'encollage 37, 38 et la feuille 1 qui se trouve sensiblement à la même température que la périphérie du tambour rotatif 4 maintenue à une température relativement basse soit par une circulation interne de liquide réfrigérant, soit simplement par l'importance de sa masse métallique. Dans ces conditions, dès leur contact avec la feuille 1 qui se déplace à grande vitesse dans le sens des flèches 2, les huit brins 8 groupés par quatre latéralement dans les deux ensembles 35 et 36 se trouvent fixés par collage à l'état tendu sur ladite feuille 1. Une différence de vitesse linéaire entre ladite feuille 1 et l'alimentation en ruban élastique multibrins 7 par le dispositif d'alimentation 11 provoque la tension désirée pour les brins 8 ainsi fixés sur la feuille 1.

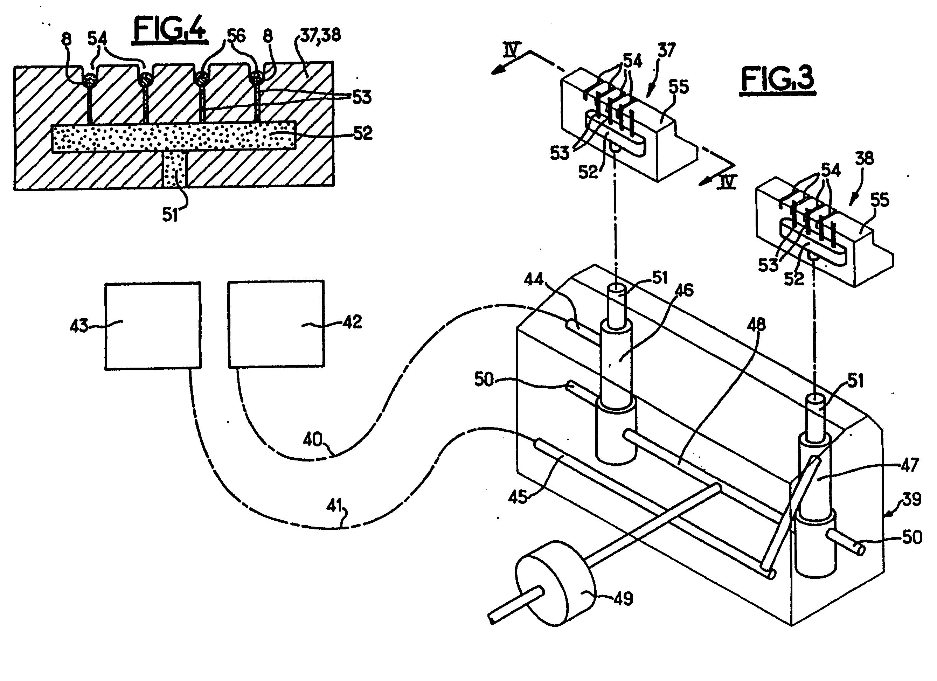

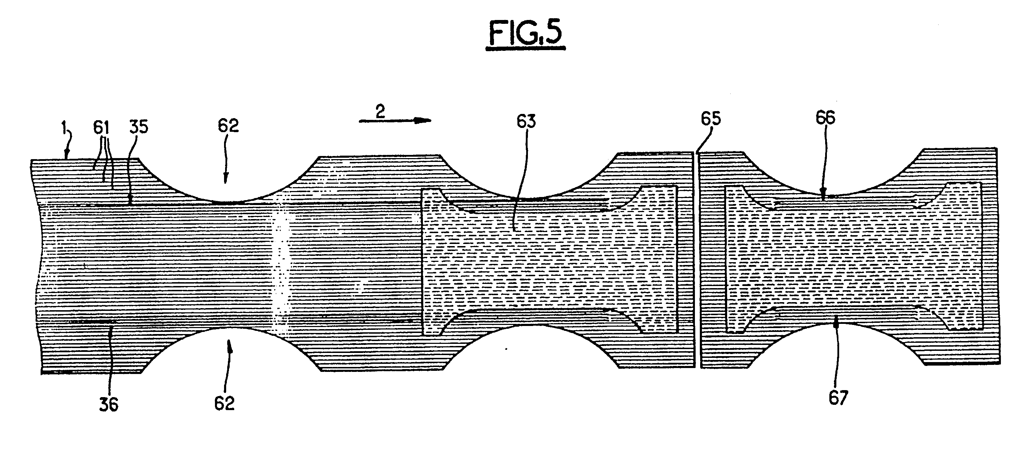

En se reportant aux fig. 3 et 4 on voit que le dispositif applicateur d'adhésif 39 comportant des résistances chauffantes non illustrées sur les figures et portant les deux blocs d'encollage 37, 38 est alimenté en adhésif liquide à haute température par les canalisations 40 et 41 reliées respectivement aux réservoirs 42 et 43 qui comportent des moyens permettant de maintenir à une température convenable l'adhésif liquide (hot met). On notera que la température habituelle d'un tel adhésif est de l'ordre de 150 _{°} C à sa sortie des réservoirs 42 et 43. Les conduits souples 40 et 41 sont reliés à des passages internes 44, 45 du dispositif applicateur d'adhésif 39 lesquels conduisent respectivement à deux organes d'extrusion 46, 47 dont la commande est efiectuée par de l'air comprimé provenant des passages 48 et alimentés par l'intermédiaire d'une électrovanne de commande 49, l'échappement de l'air comprimé se faisant par les conduits 50 qui débouchent à l'extérieur du dispositif applicateur 39.

Chaque organe d'extrusion 46, 47 communique par le passage 51 avec une chambre de distribution d'adhésif 52 qui est pratiquée à l'intérieur de chacun des blocs d'encollage 37, 38. L'adhésif ainsi extrudé dans chacune des chambres de distribution 52 peut en être extrait par intermittence par les passages calibrés 53 qui, dans l'exemple illustré sont au nombre de quatre et débouchent verticalement chacun à la partie inférieure d'une gorge longitudinale 54 horizontale pratiquée sur la face supérieure 55 de chaque bloc d'encollage 37, 38 et sur toute sa largeur.

L'adhésif à l'état liquide qui se trouve dans la chambre de distribution 52 peut alimenter chacune des gorges 54 par intermittence lorsque l'électrovanne de commande 49 est ouverte, laissant passer l'air comprimé qui est capable de chasser vers le haut l'adhésif liquide en actionnant deux distributeurs à deux voies non illustrés sur les figures et se trouvant dans chaque organe d'extrusion 46, 47 et qui mettent en communication les passages internes 44, 45 avec les passages de sortie 51. Dans cette position de l'électrovanne 49, de l'adhésif liquide 56 remplit donc la partie inférieure de chacune des gorges longitudinales 54. Dans ces conditions, chaque brin élastique 8 passant à l'intérieur de l'ume desdites gorges longitudinales 54 se trouve complètement noyé à l'intérieur de l'adhésif liquide comme on peut le voir sur la coupe de la fig. 4. A sa sortie de chaque bloc d'encollage 37, 38 chaque brin 8 se trouve donc complétement enrobé d'adhésif liquide à haute température. A la sortie des blocs d'encollage, la température de l'adhésif est de l'ordre de 120° C. Dés son contact avec la feuille souple 1, la température s'abaisse au-dessous de 100_{°} ce qui provoque la solidification de l'adhésif liquide et la fixation par collage des brins 8 à la surface de la feuille 1.

Les blocs d'encollage 37, 38 et le tambour rotatif 4 sont disposés de façon que le point de contact des brins élastiques tendus 8 enrobés d'adhésif se fasse au-dessous du niveau des gorges longitudinales 54 des blocs d'encollage 37, 38. De plus, le point de contact des brins 8 avec la feuille 1 est situé sensiblement à la moitié du parcours de la feuille 1 en contact avec le tambour 4.

On notera en outre sur la fig. 1 l'existence d'une barre transversale de relevage 57 disposée sous les deux ensembles de brins élastiques 35, 36 juste en amont du dispositif applicateur d'adhésif 39. La barre transversale 57 peut être relevée sous l'action du lever de commande 58 qui pivote autour de l'axe 59 et dont le mouvement est commandé par le vérin 60. Lors d'arrêt de l'ensemble de la machine, il est donc possible de soulever les deux ensembles de brins élastiques 35, 36 de façon que ceux-ci quittent les gorges longitudinales 54 évitant ainsi qu'ils ne soient maintenus en contact avec l'adhésif. Avant de remettre en marche la machine, la barre 57 est de nouveau abaissée, les deux ensembles de quatre brins élastiques 35, 36 reprenant leur position à l'intérieur des gorges longitudinales 54 comme illustré sur les figures 1, 3 et 4.

En se reportant à la fig. 5 on peut suivre le déroulement de quelques unes des principales étapes du procédé de fabrication de couches-culottes selon l'invention. Sur la fig. 5 on retrouve la feuille souple imperméable qui se déplace selon la flèche 2. Selon l'étape située le plus à gauche de la fig. 5, on voit que la feuille 1 a déjà reçu les deux ensembles de brins élastiques 35, 36 qui ont été fixés par collage sur certaines parties de leur longueur à la surface de la feuille 1. Ladite feuille a également reçu sur sa même surface une pluralité de lignes d'encollage longitudinales 61 paralléles et réparties sur toute sa surface. Les brins 8 sont fixés entre deux lignes d'encollage 61 parallèles.

Après cette première étape on procède à une découpe destinée à former les passages des jambes 62, cette découpe se faisant jusqu'au voisinage du brin élastique 8 le plus proche du bord latéral externe sans toutefois découper ledit brin élastique.

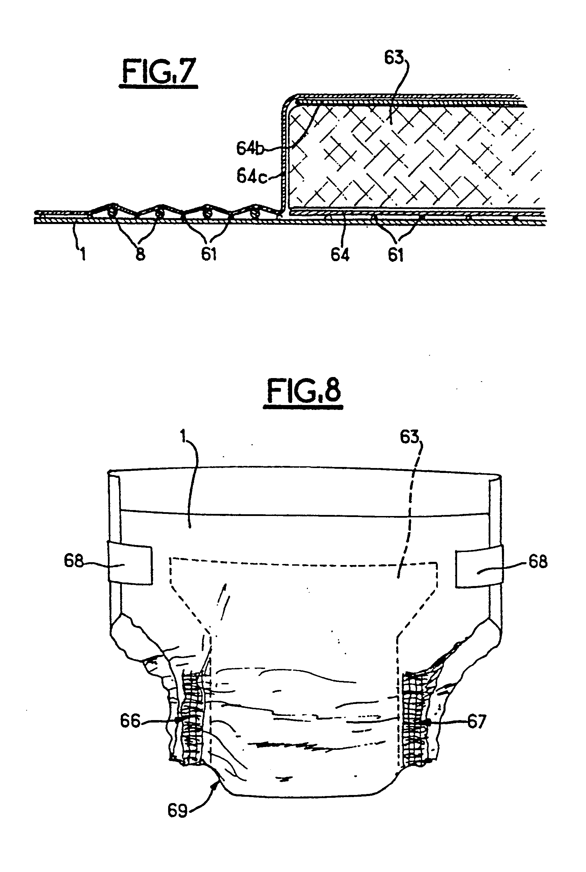

Toujours en se déplaçant vers la droite sur la fig. 5, on dépose ensuite sur la surface munie des lignes d'encollage 61 de la feuille 1, un matelas absorbant 63 qui peut éventuellement être enserré entre deux feuilles de ouate de cellulose perméable 64a et 64b et recouvert ensuite d'un voile perméable en non tissé 64c qu'on aperçoit sur la vue en coupe de la fig. 7. La fixation de ces différents éléments sur la surface de la feuille 1 se fait grâce aux lignes d'encollage longitudinales 61.

On découpe ensuite transversalement la bande continue obtenue de façon à former les différentes couches-culottes. Lors de cette opération, qui se fait selon la ligne de découpe 65, les deux ensembles de brins élastiques 35, 36 se trouvent sectionnés et se rétractent donc jusqu'au point où ils sont fixés par collage à la surface de la feuille 1 formant ainsi deux zones élastiques 66, 67 au voisinage de l'entrejambe de la couche-culotte. On notera que cette rétraction se fait pour chaque brin élastique à l'intérieur d'une gaine formée par la surface de la feuille 1 et le voile perméable 64c qui le recouvre et qui s'y trouve fixé par les lignes d'encollage parallèles 61. Pour simplifier la couche-culotte découpée illustrée à la droite de la fig. 5 est représentée à l'état tendu et sans les attaches adhésives qui permettent son utilisation.

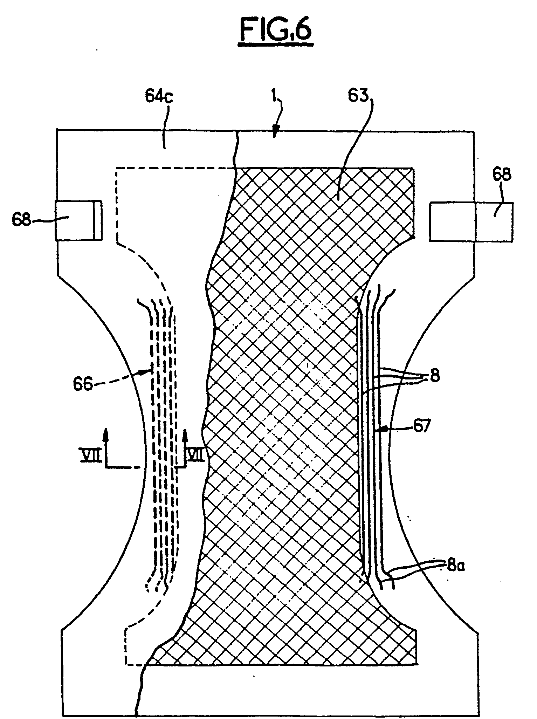

Les fig. 6 à 8 montrent une couche-culotte obtenue selon le procédé de l'invention. On retrouve sur ces figures schématiques la feuille imperméable 1 formant la culotte et les deux ensembles élastiques 66, 67 chacun formé de quatre brins parallèles 8 collés à la surface de la feuille 1 parallèlement à l'axe longitudinal de la couche-culotte au voisinage des zones de l'entrejambe, On retrouve également le matelas absorbant 63 et le voile perméable supérieur 64c qui recouvre l'ensemble. On voit également sur la fig. 6 les extrémités rétractées non encollées 8a de chaque brin élastique 8. On notera que ces extrémités sont de faible longueur compte tenu des très grandes capacités d'allongement, de l'ordre de 300% des brins élastiques 8 utilisés.

La partie arrière de la couche-culotte porte en outre deux dispositifs d'attache par adhésif 68 qui n'ont pas été représentés sur la fig. 5.

Telle que représenté sur la fig. 6, c'est-à-dire en position tendue de la couche-culotte, on voit que le brin élastique 8 de chacun des ensembles 67, 66 se trouvant le plus proche du coussin absorbant 63 est à proximité immédiate du bord latéral dudit coussin. La plus grande rigidité de la couche-culotte à l'endroit du matelas absorbant 63 et en particulier à l'endroit de chacun de ces bords latéraux entraîne une moindre capacité de déformation de la couche-culotte pour le brin élastique 8 qui se trouve le plus prés du bord latéral du matelas absorbant 63. Au contraire, plus on s'éloigne du bord latéral du matelas absorbant 63, plus le matériau de la couche-culotte est souple et plus le brin élastique peut exercer son effet de rétraction. La combinaison des brins élastiques multiples 8 mutuellement écartés avec le matelas absorbant entraîne donc par cet effet la formation d'une auge ou surface convexe vers l'extérieur.

Les deux ensembles élastiques d'entrejambe 66 et 67 exercent, lorsqu'ils sont laissés à eux-mêmes une traction sur la matière de la couche-culotte qui prend au voisinage de l'entrejambe à convexité progressive dirigée vers l'extérieur comme on le voit sur la fig. 8 une forme d'auge 69 qui permet d'améliorer son adaptation sur l'enfant en bas-êge ou l'utilisateur. De chaque côté du matelas absorbant 63 au voisinage de l'entrejambe se trouvent disposés quatre brins élastiques parallèles chacun écartés d'environ 4 à 6 mm du brin adjacent. Cette disposition se retrouve sur la vue en coupe de la fig. 7. La pression due au serrage élastique sur les cuisses est mieux répartie puisqu'elle est divisée entre plusieurs brins 8. Enfin, l'aspect extérieur de la couche-culotte se trouve amélioré par la formation d'un bord froncé de chaque côté du matelas absorbant dans la zone de l'entrejambe comme on peut le voir sur la fig. 8.

## Revendications

1. Procédé de fabrication en continu de couches-culottes à jeter comprenant un coussin absorbant posé entre une enveloppe extérieure imperméable à l'humidité et un voile intérieur perméable, l'ensemble comportant des découpe; latérale; pour permettre le passage des jambes et des moyens élastiques rectilignes fixés à l'enveloppe imperméable par collage sur au moins une partie de leur longueur et disposés de chaque côté du coussin absorbant, procédé dans lequel les moyens élastiques sont alimentés en continu, encollés de manière intermittente et mis en contact à l'état tendu avec la face interne de l'enveloppe extérieure imperméable entraînée par un tambour rotatif à surface lisse, caractérisé par le fait qu'on alimente en continu avec une tension prédéterminée un ruban élastique multibrins (7) constitué par extrusion à chaud de brins individuels (8) et solidarisation ultérieure des brins (8) dans un seul plan par contact avant refroidissement; an sépare le ruban élastique multibrins en les différents brins individuels (8) qui le constituent en le faisant passer de chaque côté de plusieurs tiges verticales successives de séparation (20, 26, 30, 31, 32, 33) disposées de manière orthogonale au trajet du ruban ; on fait passer chaque brin (8) dans une gorge longitudinale supérieure (54) d'un dispositif applicateur d'adhésif (39), ladite gorge étant alimentée par intermittence en adhésif liquide à haute température et disposée de façon que le brin (8) passant dans la gorge sait noyé sur toute sa périphérie dans l'adhésif ; et on fait entrer en contact chaque brin (8) ainsi enrobé d'adhésif par intermittence et tendu, avec l'enveloppe imperméable (1) se déplaçant à la périphérie du tambour (4).

2. Procédé de fabrication selon la revendication 1, caractérise par le faif que la feuille imperméable (1) reçoit en outre des lignes d'encollage longitudinales (61) réparties sur toute sa surface, les brins (8) étant fixés entre lesdites lignes (61) de façon à se trouver enserrés dans des gaines entre la feuille imperméable (1) et les autres éléments de la couche-culotte.

3. Procédé selon les revendications 1 ou 2, caractérisé par le fait que l'on sépare le ruban élastique multibrins en deux rubans latéraux (22, 23) en les faisant passer de chaque côté d'un organe de préséparation (20) disposé de manière orthogonale au trajet du ruban on oriente le trajet de chaque ruban latéral (22, 23) parallèlement au déplacement de l'enveloppe extérieure (1) et avec l'écartement désiré entre eux; on sépare chaque ruban latéral (22, 23) en les différents brins individuels (8) qui le constituent.

4. Procédé selon l'une quelconque des revendications précédentes, caractérisé par le fait que l'alimentation du ruban élastique (7) se fait transversalement au déplacement de la feuille imperméable (I) par un rouleau d'alimentation rotatif entrainé (12) coopérant avec un galet de friction (16) entrant en contact avec le rouleau (12) sous son propre poids.

5. Procédé selon la revendication 4, caractérisé par le fait qu'on procède à un changement de direction du ruban élastique (7) alimenté transversalement de façon à le diriger longitudinalement selon l'axe de déplacement de la feuille imperméable (1) avec un écartement correspondant à celui des moyens élastiques de la couche-culotte obtenue.

6. Procédé selon l'une quelconque des revendications précédentes, caractérisé par le fait que l'alimentation du ruban èlastique multibrins (7) est faite à partir d'un réceptacle (6) renfermant le ruban (7) lové par plans successifs sous forme d'une multitude de plis de faible longueur.

7. Procédé selon la revendication 6, caractérisé par le fait que l'alimentation du ruban multibrins (7) à partir du réceptacle (6) se fait par au moins deux renvois de guidage (9, 10) afin de régulariser le déplacement du ruban (7) en amont du dispositif d'alimentation (11).

8. Procédé selon l'une quelconque des revendications précédentes, caractérisé par le fait que le ruban élastique multibrins (7) est réalisé à l'aide de plusieurs fils en latex naturel extrudés en fils de caoutchouc nus rendus solidaires les uns des autres et pouvant être étirés jusqu'à environ 300%.

9. Procédé selon l'une quelconque des revendications précédentes, caractérisé par le fait que la superficie du tambour rotatif (4) est maintenue à une température comprise entre 30 _{°} C et 100_{°}C de façon à provoquer une prise immédiate de l'adhésif liquide enrobant les brins individuels élastiques (8).

10. Installation pour la mise en oeuvre du procédé selon l'une quelconque des revendications précédentes, comprenant des moyens d'alimentation en continu pour l'enveloppe extérieure imperméable et pour les moyens élastiques, comprenant un dispositif applicateur d'adhésif (39) présentant un bloc d'encollage (37, 38) pour chaque ensemble (35, 36) de brins individuels (8), chaque bloc d'encollage comportant un ensemble de gorges longitudinales parallèles (54) pour chacun des ensembles de brins (35, 36), les brins passant à l'interieur des gorges longitudinales (54), caractérisé par le fait que les blocs d'encollage (37, 38) sont disposés de façon que les brins élastiques (8) passent au-dessus desdits blocs d'encollage.

11. Installation selon la revendication 10, caractérisé par le fait que chaque bloc d'encollage (37, 38) présente des passages d'alimentation (53) des gorges (54) reliés chacun à une chambre de distribution d'adhésif (52).

12. Installation selon l'une quelconque des revendications 10 à 11, caractérisée par le fait que le dispositif applicateur d'adhésif (39) comprend deux organes (46, 47) d'extrusion d'adhésif liquide alimentés d'une part en adhésif liquide et d'autre part en air comprimé par l'intermédiaire d'une électrovanne de commande (49).

13. Installation selon l'une quelconques des revendications 10 à 12, caractérisée par le fait que les blocs d'encollage (37, 38) et le tambour rotatif (4) sont disposés de façon que le point de contact des brins enrobés d'adhésif se fasse au dessous du niveau, des gorges longitudinales (54) des blocs d'encollage (37, 38).

14. Installation selon la revendication 13, caractérisée par le fait que le dispositif applicateur d'adhésif (39) et le tambour rotatif (4) sort disposés de façom que le point de contact des brins individuels encollés (8) avec la feuille imperméable (1) se trouvant à la surface périphérique du tambour rotatif (4), soit situé sensiblement à la moitié du parcours de la feuille (1) en contact avec le tambour.

15. Installation selon l'une quelconque des revendications 10 à 14, caractérisée par le fait qu'elle comprend en outre une barre de levage transversale (57) disposée immédiatement en amont du dispositif applicateur d'adhésif (39) pour soulever les deux ensembles (35, 36) de brins élastiques et les extraire temporairement des gorges (54) des blocs d'encollage (37, 38).

16. Couche-culotte à jeter comprenant :
une enveloppe extérieure imperméable à l'humidité présentant des lignes d'encollage longitudinales parallèles réparties sur sa surface interne ;
un coussin absorbant posé sur la surface interne de l'enveloppe extérieure imperméable et fixé à cette dernière par les lignes d'encollage précitées ;
un voile intérieur perméable sur le coussin absorbant, également fixé à l'enveloppe imperméable par les lignes d'encollage précitées ;
l'ensemble comportant des découpes latérales pour permettre le passage des jambes ;
de chaque côté du coussin absorbant plusieurs moyens élastiques rectilignes parallèles entre eux fixés par collage à l'état tendu sur l'enveloppe imperméable, caractérisée par le fait que les moyens élastiques sont constitués par au moins quatre brins élastiques individuels fixés à l'enveloppe imperméable uniquement sur une partie de leur longueur qui correspond à la zone médiane de la couche-culotte, tandis que leurs extrémités ne sont pas fixées à l'enveloppe imperméable, les brins élastiques individuels étant écartés les uns des autres de 4 à 10 mm et de préférence d'environ 6mm, ladite partie fixée par collage des brins élastiques ayant été enrobée sur toute sa périphérie d'une matière adhésive et appliquée à l'état tendu avec un allongement de l'ordre de et au maximum de 300% sur l'enveloppe imperméable, les brins élastiques étant fixés entre lesdites lignes d'encollage à l'intérieur des gaines définies entre la feuille imperméable et les autres éléments de la couche-culotte de sorte que les extrémités des brins élastiques non encollées sont rétractées à l'intérieur desdites gaines tandis que les parties encollées assurent la rétraction de la couche-culotte dans son ensemble et la formation de fronces, dans les zones d'entrejambe, et le brin le plus proche du coussin se trouvant à proximité immédiate du bord latéral extérieur du coussin.

## Claims

1. Process for the continuous manufacture of disposable integral diapers comprising an absorbent pad placed between a moisture-impervious outer enclosure and a permeable inner veil, the whole comprising side cutouts to permit passage of the legs and rectilinear elastic means attached to the impervious enclosure by gluing over at least part of their length and arranged on each side of the absorbent pad, in which process the elastic means are fed continuously, glue-coated intermittently and placed under tension in contact with the inner face of the impervious outer enclosure driven by a smooth surfaced rotary drum, characterised in that a multistrand elastic strip (7) formed by hot extrusion of individual strands (8) and subsequent joining together of the strands (8) in a single plane by contact before cooling is fed continuously at a predetermined tension; the multistrand elastic strip is divided into individual strands (8) which constitute it by making it pass to each side of a plurality of successive separator rods (20, 26, 30, 31, 32, 33) orthogonal to the strip; each strand (8) is made to pass through an upper lengthwise groove (54) of an adhesive-applicator device (39), a liquid adhesive is applied intermittently at a high temperature to said groove which is arranged so that the strand (8) passing through the groove is immersed in the adhesive over its entire periphery; and each strand (8) thus intermittently coated with adhesive and stretched is brought into contact with the impervious enclosure (1) travelling on the periphery of the drum (4).

2. Manufacturing process according to claim 1 characterised in that the impervious sheet (1) additionally receives lengthwise glue-coating lines (61) distributed over its entire surface, the strands (8) being attached between said lines (61) so that they become clamped in sheaths between the impervious sheet (1) and the other members of the integral diaper.

3. Process according to claim 1 or claim 2 characterised in that the multistrand elastic strip is divided into two side strips (22, 23) by being made to pass on each side of a preseparation member (20) orthogonal to the path of the strips; the path of each side strip (22, 23) is oriented parallel to the travel of the outer enclosure (1) and with the required gap between them; each side strip (22, 23) is divided into the various individual strands (8) which constitute it.

4. Process according to any one of the preceding claims characterised in that the elastic strip (7) is fed transversely to the travel of the impervious sheet (1) by a driven rotary feed roll (12) cooperating with a friction roller (16) coming into contact with the roll (12) under its own weight.

5. Process according to claim 4 characterised in that the direction of the transversely fed elastic strip (7) is changed so as to direct it lengthwise along the axis of travel of the impervious sheet (1) with a gap corresponding to that of the elastic means of the integral diaper.

6. Process according to any one of the preceding claims characterised in that the multistrand elastic strip (7) is fed from a container (6) enclosing the strip (7) which is coiled in successive planes in the form of a multitude of folds of a short length.

7. Process according to claim 6 characterised in that the multistrand strip (7) is fed from the container (6) by means of least two guidance returns (9, 10) in order to regulate the travel of the strip (7) upstream of the feed device (11).

8. Process according to any one of the preceding claims characterised in that the multistrand elastic strip (7) is produced by means of several natural latex filaments extruded as bare rubber filaments joined integrally to each other and capable of being stretched to approximately 300%.

9. Process according to any one of the preceding claims characterised in that the surface of the rotary drum (4) is maintained at a temperature of between 30 _{°} C and 100°C so as to cause immediate setting of the liquid adhesive coating the individual elastic strands (8).

10. Arrangement for working of the process according to any one of the preceding claims comprising continuous feed means for the impervious outer enclosure and for the elastic means, comprising an adhesive applicator device (39) incorporating a glue-coating unit (37, 38) for each set (35, 36) of individual strands (8), each glue coating unit comprising a set of parallel lengthwise grooves (54) for each of the sets of strands (35, 36), the strands passing inside the lengthwise grooves (54), characterised in that the glue coating units (37, 38) are arranged so that the elastic strands (8) pass over said glue coating units.

11. Arrangement according to claim 10 characterised in that each glue coating unit (37, 38) has passages (53) for feeding the grooves (54) each connected to an adhesive dispensing chamber (52).

12. Arrangement according to claim 10 or claim 11 characterised in that the adhesive applicator device (39) comprises two liquid adhesive extrusion devices (46, 47) fed with liquid adhesive and with compressed air by means of a control solenoid valve (49).

13. Arrangement according to any one of claims 10 to 12 characterised in that the glue coating units (37, 38) and the rotary drum (4) are arranged so that the point of contact with the adhesive coated strands occurs below the level of the longitudinal grooves (54) in the glue coating units (37, 38).

14. Arrangement according to claim 13 characterised in that the adhesive applicator device (39) and the rotary drum (4) are arranged so that the point of contact of the individual glue coated strands (8) with the impervious sheet (1) situated on the peripheral surface of the rotary drum (4) is situated substantially midway along the path of the sheet (1) in contact with the drum.

15. Arrangement according to any one of claims 10 to 14 characterised in that it additionally comprises a transverse lifting bar (57) arranged immediately upstream of the adhesive applicator device (39) for raising the two sets (35, 36) of elastic strands and for withdrawing them temporarily from the grooves (54) in the glue coating units (37, 38).

16. Disposable integral diaper comprising:
a moisture impervious outer enclosure having parallel longitudinal glue coating lines on its inside surface;
an absorbent pad disposed on and attached to the inside surface of the moisture impermeable outer enclosure by the aforementioned glue coating lines;
a pervious inner veil over the absorbent pad and also attached to the impervious enclosure by the aforementioned glue coating lines;
the whole comprising side cutouts to permit passage of the legs;
on each side of the absorbent pad a plurality of parallel rectilinear elastic means attached to the impervious enclosure under tension by gluing,
characterised in that the elastic means comprise at least four individual elastic strands attached to the impervious enclosure over only a part of their length corresponding to the median area of the diaper while their ends are not attached to the impervious envelope, the individual elastic strands being separated from each other by 4 mm to 10 mm and preferably approximately 6 mm, said part of the elastic strands attached by gluing having been coated all over with an adhesive and applied to the impervious envelope under tension with an elongation in the order of and not more than 300%, the elastic strands being attached between said glue coating lines inside sheaths defined between the impermeable sheet and the other components of the diaper so that the unglued ends of the elastic strands are retracted inside said sheaths and the glued parts cause retraction of the diaper as a whole and the formation of folds in the area between the legs, and the strand nearest the pad being in the immediate vicinity of the outside edge of the pad.

## Patentansprüche

1. Verfahren zum kontinuierlichen Herstellen von Wegwerfwindelhöschen, mit einem saugfähigen Kissen, das zwischen einer feuchtigkeitsundurchlässigen Außenhülle und einem durchlässigen Innentuch angeordnet ist, wobei die Anordnung seitliche Ausschnitte für den Durchtritt von Beinen und gerade, elastische Mittel, die an der undurchlässigen Hülle durch Kleben auf wenigstens einem Teil ihrer Länge befestigt und die auf beiden Seiten des saugfähigen Kissens angeordnet sind, aufweist, bei welchem Verfahren die elastischen Mittel kontinuierlich zugeführt, abschnittsweise mit Klebstoff beschichtet und in gespanntem Zustand mit der Innenseite der undurchlässigen Außenhülle, die von einer Drehtrommel mit glatter Fläche angetrieben wird, in Berührung gebracht werden, dadurch gekennzeichnet, daß man ein mehrfädiges, elastisches Band (7), das durch Extrusion von einzelnen Fäden (8) in der Wärme und nachfolgendes Verbinden der Fäden (8) in einer Ebene durch Berühren vor dem Abkühlen gebildet wird, kontinuierlich und mit vorgegebener Spannung zuführt, daß man das mehrfädige, elastische Band in die verschiedenen einzelnen Fäden (8), die es bilden, teilt, indem man es auf beiden Seiten mehrerer vertikaler, aufeinanderfolgender Trennstifte (20, 26, 30, 31, 32, 33), die im rechten Winkel zur Bewegungsbahn des Bandes ausgerichtet sind, vorbeibewegt, daß man jeden Faden (8) in einer oberen Längsnut (39) einer Vorrichtung (39) zum Auftragen von Klebstoff bewegt, wobei die genannte Nut absatzweise mit bei hoher Temperatur flüssigem Klebstoff derart beschickt wird und so angeordnet ist, daß der sich in der Nut bewegende Faden (8) mit seiner ganzen Umfangsfläche in Klebstoff eingetaucht ist, und daß man jeden Faden (8), der so abschnittsweise mit Klebstoff umhüllt und gespannt ist, mit der undurchlässigen Hülle (1), die sich auf der Umfangsfläche der Trommel (4) bewegt, in Berührung bringt.

2. Verfahren zum Herstellen nach Anspruch 1, dadurch gekennzeichnet, daß die undurchlässige Lage (1) überdies längliche Klebstoffstriche (61), die über ihre ganze Fläche verteilt sind, erhält, wobei die Fäden (8) zwischen diesen Linien (61) so befestigt werden, daß sie in den Hohlräumen zwischen der undurchlässigen Lage (1) und den anderen Teilen des Windelhöschens eingeschlossen sind.

3. Verfahren nach den Ansprüchen 1 oder 2, dadurch gekennzeichnet, daß man das mehrfädige, elastische Band in zwei seitliche Bänder (22, 23) teilt, indem man diese auf beiden Seiten eines Vortrennorganes (20) vorbeiführt, das in rechtem Winkel zur Bewegungsbahn des Bandes ausgerichtet ist, daß man die Bahn jedes seitlichen Bandes (22, 23) parallel zur Bewegung der Außenhülle (1) und mit dem gewünschten Abstand zwischen ihnen ausrichtet, daß man jedes seitliche Band (22, 23) in die es bildenden, verschiedenen, einzelnen Fäden (8) teilt.

4. Verfahren nach einem der vorhergehenden Ansprüche, dadurch gekennzeichnet, daß das Zuführen des elastischen Bandes (7) quer zur Bewegung der undurchlässigen Lage (1) durch eine drehangetriebene Zuführrolle (12) erfolgt, die mit einer Reibrolle (16) zusammenwirkt, die mit der Rolle (12) unter ihrem Eigengewicht in Berührung tritt.

5. Verfahren nach Anspruch 4, dadurch gekennzeichnet, daß man die Richtung des elastischen Bandes (7), das quer zugeführt wird, ändert, um es längs der Bewegungsachse der undurchlässigen Hülle (1) mit einem Abstand entsprechend dem der elastischen Mittel des erhaltenen Windelhöschens auszurichten.

6. Verfahren nach einem der vorhergehenden Ansprüche, dadurch gekennzeichnet, daß das Zuführen des elastischen mehrfädigen Bandes (7) ausgehend von einem Aufnahmebehälter (6) erfolgt, der das in aufeinanderfolgenden Lagen in Form einer Vielzahl von Falten mit kurzer Länge abgelegte Band (7) enthält.

7. Verfahren nach Anspruch 6, dadurch gekennzeichnet, daß das Zuführen des mehrfädigen Bandes (7) vom Aufnahmebehälter (6) über wenigstens zwei Umlenkführungen (9, 10) erfolgt, um den Vorschub des Bandes (7) vor der Zuführvorrichtung (11) zu regeln.

8. Verfahren nach einem der vorhergehenden Ansprüche, dadurch gekennzeichnet, daß das elastische, mehrfädige Band (7) aus mehreren Naturlatexfäden besteht, die als nackte Kautschukfäden extrudiert und miteinander verbunden werden und die bis zu etwa 300 % gedehnt werden können.

9. Verfahren nach einem der vorhergehenden Ansprüche, dadurch gekennzeichnet, daß die Mantelfläche der Drehtrommel (4) auf einer Temperatur zwischen 30 _{°} C und 100_{°}C gehalten wird, um ein unmittelbares Anziehen des flüssigen Klebstoffes, der die einzelnen, elastischen Fäden (8) einhüllt, zu bewirken.

10. Einrichtung zum Durchführen des Verfahrens nach einem der vorhergehenden Ansprüche, mit Mitteln zum kontinuierlichen Zuführen der undurchlässigen Außenhülle und der elastischen Mittel, mit einer Vorrichtung (39) zum Auftragen von Klebstoff, die einen Block (37, 38) zum Auftragen von Klebstoff für jede Gruppe (35, 36) von einzelnen Fäden (8) aufweist, wobei jeder Block zum Auftragen von Klebstoff eine Gruppe von parallelen, längslaufenden Nuten (54) für jede Gruppe der Fäden (35, 36) besitzt, wobei sich die elastischen Fäden durch den Innenraum der längslaufenden Nuten (54) bewegen, dadurch gekennzeichnet, daß die Blöcke zum Auftragen von Klebstoff (37, 38) so angeordnet sind, daß sich die elastischen Fäden (8) über den genannten Blökken zum Auftragen von Klebstoff bewegen.

11. Einrichtung nach Anspruch 10, dadurch gekennzeichnet, daß jeder Block (37, 38) zum Auftragen von Klebstoff Kanäle (53) für das Speisen der Nuten (54) aufweist, die jeweils mit einer Kammer (52) zum Verteilen von Klebstoff verbunden sind.

12. Einrichtung nach einem der Ansprüche 10 bis 11, dadurch gekennzeichnet, daß die Vorrichtung (39) zum Auftragen von Klebstoff zwei Organe (46, 47) für das Extrudieren von flüssigem Klebstoff aufweist, die über ein Elektroregelventil (49) einerseits mit flüssigem Klebstoff und anderseits mit Druckluft gespeist werden.

13. Einrichtung nach einem der Ansprüche 10 bis 12, dadurch gekennzeichnet, daß die Blöcke (37, 38) zum Auftragen von Klebstoff und die Drehtrommel (4) so angeordnet sind, daß der Punkt der Berührung der mit Klebstoff umhüllten Fäden unter dem Niveau der längslaufenden Nuten (54) der Blöcke (37, 38) zum Auftragen von Klebstoff zu liegen kommt.

14. Einrichtung nach Anspruch 13, dadurch gekennzeichnet, daß die Vorrichtung (39) zum Auftragen von Klebstoff und die Drehtrommel (4) so angeordnet sind, daß der Punkt der Berührung der einzelnen, mit Klebstoff beschichteten Fäden (8) mit der undurchlässigen Hülle (1), die sich auf der Mantelfläche der Drehtrommel (4) befindet, im wesentlichen in der Mitte des Weges der Hülle (1) in Berührung mit der Trommel liegt.

15. Einrichtung nach einem der Ansprüche 10 bis 14, dadurch gekennzeichnet, daß sie überdies eine Querhebelstange (57) besitzt, die unmittelbar vor der Vorrichtung (39) zum Auftragen von Klebstoff angeordnet ist, um die beiden Gruppen (35, 36) der elastischen Fäden anzuheben und sie zeitweise aus den Nuten (54) der Blöcke (37, 38) zum Auftragen von Klebstoff herausziehen.

16. Wegwerfwindelhöschen
mit einer feuchtigkeitsundurchlässigen Außenhülle, die auf ihrer Innenseite verteilt, längslaufende Klebstoffstriche aufweist;
mit einem saugfähigen Kissen, das auf der Innenseite der undurchlässigen Außenhülle angeordnet und an letzterer durch die genannten Klebstoffstriche befestigt ist;
mit einem durchlässigen Innentuch auf dem saugfähigen Kissen, das auf der undurchlässigen Außenhülle ebenfalls durch die genannten Klebstoffstriche befestigt ist;
wobei die Anordnung seitliche Ausschnitte für den Durchtritt von Beinen aufweist;
und mit mehreren, geraden, zueinander parallelen, elastischen Mitteln, die auf beiden Seiten des saugfähigen Kissens durch Kleben in gespanntem Zustand auf der undurchlässigen Außenhülle befestigt sind, dadurch gekennzeichnet, daß die elastischen Mittel von wenigstens vier einzelnen, elastischen Fäden gebildet werden, die an der undurchlässigen Außenhülle nur über einen Teil ihrer Länge befestigt sind, der dem mittleren Bereich des Windelhöschens entspricht, wogegen ihre Enden nicht an der undurchlässigen Hülle befestigt sind, daß die einzelnen, elastischen Fäden voneinander einen Abstand von 4 bis 10 mm und vorzugsweise von etwa 6 mm aufweisen, daß der durch Kleben befestigte Abschnitt der elastischen Fäden auf seinem ganzen Umfang mit einem Klebstoff umgeben und in gespanntem Zustand mit einer Dehnung in der Größenordnung von höchstens 300 % auf der undurchlässigen Außenhülle befestigt worden ist, daß die elastischen Fäden zwischen den genannten Klebstofflinien im Inneren von Hohlräumen angeordnet sind, die zwischen der undurchlässigen Lage und den anderen Bestandteilen des Windelhöschens gebildet sind, so daß die nicht mit Klebstoff beschichteten Enden der elastischen Fäden in das Innere der genannten Hohlräume zurückgezogen werden, wogegen die mit Klebstoff beschichteten Teile das Zusammenziehen des Windelhöschens in seiner Gesamtheit und die Bildung von Falten im Schrittbereich gewährleisten und daß sich der Faden, der dem Kissen am nächsten angeordnet ist, in unmittelbarer Nähe des Seitenrandes des Kissens befindet.
